# EUROPÄISCHE PATENTSCHRIFT

(11) **EP 0 412 283 B1**
(45) Veröffentlichungstag und Bekanntmachung des Hinweises auf die Patenterteilung: **01.12.1993**
(21) Anmeldenummer: 90112425.5
(22) Anmeldetag: 29.06.1990
(51) Int. Cl.: A61M 5/28

(54) **Spritzenzylinder für medizinische Zwecke**
Syringe cylinder for medical use
Cylindre de seringue à usage médical

(30) Priorität: 27.07.1989 DE 3924830
(43) Veröffentlichungstag der Anmeldung: 13.02.1991
(73) Patentinhaber: Arzneimittel GmbH Apotheker Vetter & Co. Ravensburg, D-88212 Ravensburg (DE)
(72) Erfinder: Vetter, Helmut, 7980 Ravensburg (DE); Geprägs, Peter, 7987 Weingarten (DE)
(74) Vertreter: Fay, Hermann, Dipl.-Phys. Dr.

(56) Entgegenhaltungen:
- AU-B- 491 860
- CH-A- 575 765
- DE-A- 1 491 688
- DE-A- 2 258 371
- DE-A- 2 617 017
- DE-C- 577 364
- US-A- 1 803 316
- US-A- 2 368 431

## Beschreibung

Die Erfindung betrifft einen Spritzenzylinder für medizinische Zwecke mit einem verschiebbaren Spritzenkolben, wobei der Spritzenzylinder als zylindrisches Rohr ausgebildet ist, das an seinem kanülenseiten Ende an seiner Mantelfläche mit einem ringförmigen Bund versehen ist, der einen Rastsitz für ein kanülenseitiges Verschlußteil bildet, wobei das Verschlußteil als Durchstechstopfen ausgebildet ist, der an seiner dem Spritzenkolben gegenüberstehenden Stirnfläche mit einer sackartigen Ausnehmung versehen ist, zwischen deren Boden und der kanülenseitigen Stirnfläche eine durchstechbare Trennwand gebildet ist.

Solche Spritzenzylinder sind in der Praxis bekannt und beispielsweise in der US-A-1 803 316 beschrieben. Dort ist der Spritzenzylinder mit der Injektionssubstanz fertig abgefüllt, so daß sich gegenüber einer Abfüllung der Injektionssubstanz in Ampullen oder Vials der Vorteil ergibt, daß eine Überführung der Substanz vor der Applikation in das Injektionssystem entfällt. Der Spritzenzylinder wird in ein Applikationsteil eingesetzt, auf das ein Nadelansatzteil aufsetzbar ist. Das Applikationsteil ist mit einer Kolbenstange versehen, über die der Spritzenkolben verstellbar ist.

Um das kanülenseitige Verschlußteil sicher am Spritzenzylinder zu halten, wenn die zu injizierende Substanz unter erhöhtem Druck aus dem Spritzenzylinder herausgepreßt wird, ist am Verschlußteil ein radial nach außen vorstehender Ringbund vorgesehen, der in eine Ringnut des Spritzenzylinders greift.

In der deutschen Patentschrift DE-C-577 364 ist eine Ampulle beschrieben, bei der der Ampullenzylinder einen radial einwärts vorstehenden Bund aufweist, der in eine Ringnut des Durchstechstopfens greift. Auch diese Ausgestaltung dient dazu, das Verschlußteil fest in dem Ampullenzylinder zu halten.

Schließlich ist aus der DE-A-2 258 371 eine aus Kunststoff und Glas bestehende Spritzenanordnung bekannt, bei der der Spritzenzylinder jeweils mit einem im Querschnitt näherungsweise kreisförmigen Ringflansch versehen ist. Dieser Ringflansch dient als Rastvorsprung für das jeweils endseitig aufzusetzende Nadelansatzteil bzw. die Fingerauflage.

Bei all diesen bekannten Spritzenzylindern ist der Innnenraum nach dem Ansetzen des Verschlußteils bei schon eingesetztem Spritzenkolben hermetisch gegenüber dem Außenraum abgeschlossen. Die schon eingebrachte Injektionssustanz kann dann keinem weiteren Verfahrensschritt mehr unterworfen werden, insbesondere ist die Lyophilisierung der Injektionssubstanz in derartigen Spritzenzylindern nicht möglich.

Da es sich bei diesen Spritzenzylindern, wie bei den Ampullen auch, um Wegwerf-Produkte handelt, die im übrigen in hoher Stückzahl benötigt werden, müssen diese einfach und kostengünstig herstellbar und befüllbar sein.

Der Erfindung liegt die Aufgabe zugrunde, einen Spritzenzylinder der eingangs genannten Art so auszubilden, daß er mit minimalen Kosten herstellbar, dabei in Großserien einfach und schnell zu befüllen ist und nach dem Befüllen eine Lyophilisierung des Füllguts erfolgen kann, zugleich aber der Einsatz als vollwertiger Spritzenzylinder unmittelbar bei der Applikation der Injektionssubstanz möglich ist.

Diese Aufgabe wird nach der Erfindung dadurch gelöst, daß der Durchstechstopfen mit zumindest einer, von der sackartigen Ausnehmung radial nach außen zur Mantelfläche sich erstreckenden Bohrung versehen ist.

Der durch die Erfindung erreichte Fortschritt besteht im wesentlichen darin, daß bei zunächst nur teilweise in den Spritzenzylinder eingesetzem Verschlußteil über die Ausnehmung und die Bohrung das Lösungsmittel aus dem Inneren des Spritzenzylinders abgesaugt und anschließend das Verschlußteil in seine vorgesehene Position verschoben werden kann. In der vorgesehenen Position des Verschlußteils sind dann die Bohrung bzw. die Bohrungen durch die Innenfläche des Spritzenzylinders hermetisch verschlossen. Anschließend ist der Spritzenzylinder nach Anbringung eines Nadelansatzstücks und gegebenenfalls einer Fingerauflage unmittelbar zur Applikation der in ihm enthaltenen Injektionssustanz einsetzbar.

Um den Halt des Verschlußteils während der Lyophilisierung zu verbessern, kann der Durchstechstopfen im Randbereich seiner der Trennwand abgewandten Stirnseite umfangsseitig mit einer keilförmigen Nut versehen sein, in die der am Spritzenzylinder endseitig angeordnete Ringflansch greift.

In einer vorteilhaften Ausgestaltung der Erfindung besteht die Möglichkeit, in der sackartigen Ausnehmung eine Filterscheibe anzuordnen, die senkrecht zur Längsachse ausgerichtet ist und in einer Ringnut gehalten ist. Diese Filterscheibe verhindert bei der Applikation der zu injizierenden Substanz, daß auch nicht oder sich nur schwer lösende Bestandteile aus dem Spritzenzylinder zur Kanüle gelangen.

Schließlich kann an der Außenmantelfläche des Spritzenzylinders eine in Umfangsrichtung verlaufende Rastnut zum Anschluß eines stirnseitig aufgesetzten Nadelansatzstückes vorgesehen sein, wobei das hohlzylindrisch ausgebildete Nadelansatzstück mit einer in die Rastnut greifenden Ringleiste versehen ist.

Im folgenden wird die Erfindung an einem in der Zeichnung dargestellten Ausführungsbeispiel näher erläutert; es zeigen:
- Fig. 1: den Spritzenzylinder im Längsschnitt,
- Fig. 2: die Anordnung des Verschlußteils während der Lyophilisierung,
- Fig. 3: eine Detaildarstellung des Gegenstands nach Fig. 1, jedoch mit aufgesetztem Nadelansatzstück.

Der in der Zeichnung dargestellte Spritzenzylinder 1 ist für medizinische Zwecke vorgesehen und weist einen verschiebbaren Spritzenkolben 2 sowie ein kanülenseitiges Verschlußteil 3 auf.

Der Spritzenzylinder 1 ist als zylindrisches Rohr 4 ausgebildet, das an seinem kanülenseitigen Ende 4.1 an seiner Innenmantelfläche mit einem ringförmigen Bund 5 versehen ist, der einen Rastsitz für das Verschlußteil 3 bildet.

Das Verschlußteil 3 ist als Durchstechstopfen ausgebildet, der an seiner dem Spritzenkolben 1 gegenüber stehenden Stirnfläche mit einer sackartigen Ausnehmung 6 versehen ist. Zwischen dem Boden 6.1 dieser Ausnehmung 6 und der kanülenseitigen Stirnfläche 3.1 ist hierdurch eine durchstechbare Trennwand 3.2 gebildet. Dazu ist die sackartige Ausnehmung 6 von einer zylindrischen Bohrung mit einer ebenen, zur Längsachse senkrechten Bodenfläche 6.1 gebildet.

Der ringförmige Bund 5 ist von einem radial einwärts vorstehenden Wulst gebildet, der in eine der Ringnuten des Durchstechstopfens greift. Hierdurch wird sichergestellt, daß der Durchstechstopfen seine vorgesehene Lage im Spritzenzylinder 1 einnimmt, so daß beim Aufsetzen beispielsweise einer Kanüle 7 die Trennwand 3.2 auch sicher durchstochen werden kann, und daß der Durchstechstopfen darüber hinaus in dieser Lage bis zur Applikation gehalten wird. Der Spritzenzylinder 1 ist an seinen beiden Stirnseiten 4.1, 4.2 jeweils mit einem im Querschnitt näherungsweise kreisförmigen Ringflansch 8 versehen, wobei der Durchstechstopfen mit dem Rand seiner kanülenseitigen Stirnfläche 3.1 diesem Ringflansch 8 anliegt. Auf diese Weise erhält der Durchstechstopfen in seiner vorgesehenen Lage noch besseren Halt.

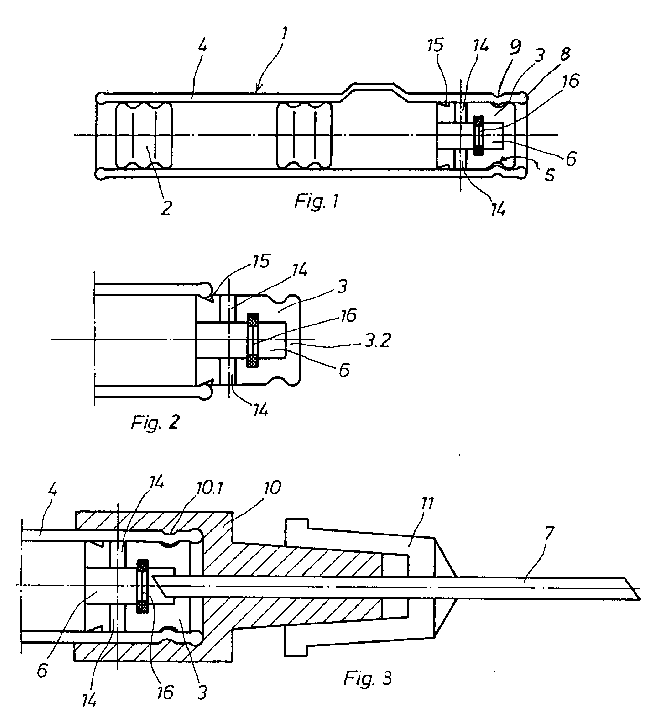

An der Außenmantelfläche des Spritzenzylinders 1 ist eine in Umfangsrichtung verlaufende Rastnut 9 zum Anschluß eines stirnseitig aufgesetzten Nadelansatzstücks 10 vorgesehen, wobei das hohlzylindrisch ausgebildete Nadelansatzstück 10, wie sich dies insbes. aus Fig. 3 ergibt, mit einer in die Rastnut 9 greifenden Ringleiste 10.1 versehen ist. Auf das Nadelansatzstück 10 ist die mit einer Kappe 11 versehene Kanüle 7 aufgesetzt, deren eines Ende sich vollständig durch eine Bohrung im Nadelansatzstück 10 hindurch erstreckt und so ausgebildet ist, daß es die Trennwand 3.2 des Verschlußteils 3 beim Aufsetzen durchsticht. Dabei besteht jedoch auch die nicht näher dargestellte Möglichkeit, daß der Spritzenzylinder 1 bereits mit einem Nadelansatzstück 10 fertig konfektioniert ist, auf das ein Tip-Cap aufgesetzt ist. Der Verschlußstift des Tip-Cap kann sich dann durch das Nadelansatzstück 10 sowie die schon geöffnete Trennwand 3.2 hindurch erstrecken.

An dem dem Verschlußteil 3 abgewandten Ende des Spritzenzylinders 1 kann in ebenfalls nicht näher dargestellter Weise eine Fingerauflage aufgerastet werden, wofür eine an sich bekannte Luer-Aufprellkappe Verwendung finden kann.

Für die Lyophilisierung unmittelbar im Spritzenzylinder 1 ist der Durchstechstopfen mit zwei von der sackartigen Ausnehmung 6 radial nach außen zur Mantelfläche sich erstreckenden Bohrungen 14 versehen. Wie sich im einzelnen aus der Fig. 2 ergibt, ist der Durchstechstopfen im Randbereich seiner der Trennwand 3.2 abgewandten Stirnseite umfangsseitig mit einer keilförmigen Nut 15 versehen, die zur Aufnahme des stirnseitig am Spritzenzylinder 1 angeordneten Ringflansches 8 vorgesehen ist. Auf diese Weise wird das Verschlußteil 3 während der Lyophilisierung in der in Fig. 2 dargestellten Stellung gehalten, bis das gesamte Lösungsmittel durch die Bohrungen 14 aus dem Inneren des Spritzenzylinders 1 entfernt ist. Anschließend wird der Durchstechstopfen in seine vorgesehene Stellung in den Spritzenzylinder 1 eingedrückt, wodurch sich die Bohrungen 14 über die Innenmantelfläche des Spritzenzylinders selbsttätig schließen.

Um bei der Applikation der zu injizierenden Substanz zu verhindern, daß auch nicht oder sich nur schwer lösende Bestandteile oder Rückstände aus dem Spritzenzylinder 1 zur Kanüle 7 gelangen, ist in der sackartigen Ausnehmung 6 eine Filterscheibe 16 angeordnet, die senkrecht zur Längsachse ausgerichtet und in einer Ringnut gehalten ist.

Diese Filterscheibe 16 ist, soweit der Durchstechstopfen für die Lyophilisierung eingerichtet ist, zwischen den Bohrungen 14 und der Trennwand 3.2 angeordnet.

## Patentansprüche

1. Spritzenzylinder für medizinische Zwecke, mit einem verschiebbaren Spritzenkolben, wobei der Spritzenzylinder (1) als zylindrisches Rohr (4) ausgebildet ist, das an seinem kanülenseitigen Ende (4.1) an seiner Mantelfläche mit einem ringförmigen Bund (5) versehen ist, der einen Rastsitz für ein kanülenseitiges Verschlußteil (3) bildet, wobei das Verschlußteil (3) als Durchstechstopfen ausgebildet ist, der an seiner dem Spritzenkolben (2) gegenüberstehenden Stirnfläche mit einer sackartigen Ausnehmung (6) versehen ist, zwischen deren Boden (6.1) und der kanülenseitigen Stirnfläche (3.1) eine durchstechbare Trennwand (3.2) gebildet ist, dadurch gekennzeichnet, daß der Durchstechstopfen mit zumindest einer, von der sackartigen Ausnehmung (6) radial nach außen zur Mantelfläche sich erstreckenden Bohrung (14) versehen ist.

2. Spritzenzylinder nach Anspruch 1, dadurch gekennzeichnet, daß der Durchstechstopfen im Randbereich seiner der Trennwand (3.2) abgewandten Stirnseite umfangsseitig mit einer keilförmigen Nut (15) versehen ist.

3. Spritzenzylinder nach Anspruch 1 oder 2, dadurch gekennzeichnet, daß in der sackartigen Ausnehmung (6) eine Filterscheibe (16) angeordnet ist, die senkrecht zur Längsachse ausgerichtet und in einer Ringnut gehalten ist.

4. Spritzenzylinder nach einem der Ansprüche 1 bis 3, dadurch gekennzeichnet, daß an seiner Außenmantelfläche eine in Umfangsrichtung verlaufende Rastnut (9) zum Anschluß eines stirnseitig aufgesetzten Nadelansatzstückes (10) vorgesehen ist, wobei das hohlzylindrisch ausgebildete Nadelansatzstück (10) mit einer in die Rastnut (9) greifenden Ringleiste (10.1) versehen ist.

## Claims

1. A syringe cylinder for medical purposes, comprising a displaceable syringe plunger, wherein the syringe cylinder (1) is in the form of a cylindrical tube (4) which at its cannula end (4.1) is provided at its peripheral surface with an annular shoulder (5) which forms a retaining seat for a closure member (3) at the cannula end, wherein the closure member (3) is in the form of a pierceable plug which at its end face opposite the syringe plunger (2) is provided with a blind recess (6), between the bottan (6.1) of which and the end face (3.1) that is towards the cannula is formed a pierceable partition wall (3.2), characterised in that the pierceable plug is provided with at least one bore (14) which extends from the blind opening (6) radially outwardly to the peripheral surface.

2. A syringe cylinder according to claim 1 characterised in that the pierceable plug is provided at its periphery with a wedge-shaped groove (15) in the edge region of the end of the pierceable plug, which is remote from the partition wall (3.2).

3. A syringe cylinder according to claim 1 or claim 2 characterised in that disposed in the blind opening (6) is a filter disc (16) which is oriented perpendicularly to the longitudinal axis and held in an annular groove.

4. A syringe cylinder according to one of claims 1 to 3 characterised in that provided at its outside peripheral surface is a peripherally extending retaining groove (9) for the connection of a needle attachment portion (10) which is fitted at the end thereof, wherein the needle attachment portion (10) which is of a hollow cylindrical configuration is provided with an annular ridge (10.1) which engages into the retaining groove (9).

## Revendications

1. Cylindre de seringue pour usage médical, comprenant un piston de seringue mobile, le cylindre de seringue (1) étant conformé en tube cylindrique (4) dont l'extrémité (4.1) du côté canule est munie sur sa face latérale d'un collet annulaire (5) qui forme un ajustement à encliquetage pour un élément de fermeture (3) situé du côté canule, ledit élément de fermeture (3) étant réalisé sous la forme d'un bouchon perforable et muni sur sa face frontale opposée au piston de seringue (2), d'un évidement (6) en forme de sac entre le fond (6.1) et la face frontale (3.1) du côté canule duquel est conformée une cloison (3.2) perforable, c a r a c t é r i s é e n c e que le bouchon perforable présente au moins un alésage (14) qui s'étend de l'évidement (6) en forme de sac radialement vers l'extérieur en direction de la surface latérale.

2. Cylindre de seringue selon la revendication 1, caractérisé en ce que le bouchon perforable est muni, dans la zone latérale de sa face frontale détournée de la cloison (3.2), d'une rainure circonférentielle (15) en forme de coin.

3. Cylindre de seringue selon l'une des revendications 1 ou 2, caractérisé en ce que dans l'évidement (6) en forme de sac est disposé un disque de filtre (16) qui est orienté perpendiculairement à l'axe longitudinal et maintenu dans une rainure annulaire.

4. Cylindre de seringue selon l'une des revendications 1 à 3, caractérisé en ce que sur sa surface latérale extérieure est prévue une rainure d'arrêt (9) circonférentielle pour le raccordement d'un adaptateur d'aiguille (10) emboîté du côté frontal, l'adaptateur d'aiguille (10) conformé en cylindre creux étant muni d'une baguette annulaire (10.1) qui s'engage dans la rainure d'arrêt (9).
